# EUROPEAN PATENT APPLICATION

(11) **EP 2 353 495 A1**
(43) Date of publication of application: **10.08.2011**
(21) Application number: 09830260.7
(22) Date of filing: 14.10.2009
(51) Int. Cl.: A61B 3/024, A61B 3/02, A61B 3/08

(54) **MULTIFUNCTION OPHTHALMIC EXAMINATION APPARATUS**

(30) Priority: 02.12.2008 JP 2008307539
(71) Applicant: Panasonic Electric Works Co., Ltd., Kadoma-shi Osaka 571-8686 (JP)
(72) Inventor: KOIWA, Hiroko, Kadoma-shi Osaka 571-8686 (JP); KAWAMURA, Ryo, Kadoma-shi Osaka 571-8686 (JP)
(74) Representative: Appelt, Christian W.
(86) International application number: PCT/JP2009/067788
(87) International publication number: WO 2010/064492

(57) **Abstract**

A configuration is adopted, which includes: a shallow-bottom hemispherical screen 1B and a deep-bottom hemispherical screen 1A, which are different in depth from each other. The deep-bottom hemispherical screen 1A belongs to an entirety of a hemispherical screen 1 composed of the shallow-bottom hemispherical screen 1B and a peripheral edge portion of the hemispherical screen 1. In accordance with a type of an implemented ophthalmic test, a multifunctional ophthalmic test device drives the deep-bottom hemispherical screen 1A or the shallow-bottom hemispherical screen 1B so that either of the deep-bottom hemispherical screen 1A and the shallow-bottom hemispherical screen 1B can be located at a position onto which an ophthalmic test image is projected by a projector 2. In this state, the projector 2 projects the ophthalmic test image onto either of the deep-bottom hemispherical screen 1A and the shallow-bottom hemispherical screen 1B. In such a way, a multifunctional ophthalmic test device is provided, which is capable of performing various types of ophthalmic tests by using a hemispherical screen.

## Description

### TECHNICAL FIELD

The present invention relates to a multifunctional ophthalmic test device capable of performing plural types of tests as ophthalmic tests.

### BACKGROUND ART

As ophthalmic tests at present, approximately 18 types are present, and heretofore, as a technology capable of performing the plurality of ophthalmic tests by a single device, the one described in the following Patent Literature 1 has been known. An ophthalmic test device described in this Patent Literature 1 has an object to measure vision and a visual field or retina sensitivity by the same device. Specifically, an ophthalmic test device is described, which is capable of measuring night vision and a dark-adapted visual function, dynamic vision and the retina sensitivity.

### Prior Art Literatures

### Patent Literatures

Patent Literature 1: Japanese Patent Laid-Open Publication No. 2003-93344

### DISCLOSURE OF THE INVENTION

### Problems to be Solved by the Invention

However, in the above-mentioned ophthalmic test device, the individual ophthalmic tests cannot be performed by the existing device configuration. For example, in the test device of the visual field/vision in Patent Literature 1, a dome-type screen (hemispherical screen) that is general in a visual field test cannot be used. Hence, in the above-mentioned ophthalmic test device, a range of the visual field has been measured on a planar plate. Therefore, in order to measure a wide visual field, it has been forced to adopt a mode of an observation hole, which uses an optical system. Moreover, also with regard to a vision test, owing to a restriction on device design, which is caused by adopting the mode of the observation hole, a lens for converging focal points of right and left eyes to an index mark has been required. Furthermore, with regard to a binocular vision test, the hemispherical screen cannot be used owing to reasons of halation, unevenness in brightness and the like on end portions of the screen, which occur from shape characteristics of the hemispherical screen.

In this connection, the present invention has been proposed in consideration of the above-mentioned actual circumstances. It is an object of the present invention to provide a multifunctional ophthalmic test device capable of performing various types of ophthalmic tests by using the hemispherical screen.

### Means for Solving the Problems

In order to solve the above-mentioned problems, in the present invention, a deep-bottom hemispherical screen and a shallow-bottom hemispherical screen, which are different in depth from each other, are prepared in advance, and in accordance with a type of an implemented ophthalmic test, the deep-bottom hemispherical screen or the sallow-bottom hemispherical screen is driven so that an ophthalmic test image can be projected onto either of the deep-bottom hemispherical screen and the shallow-bottom hemispherical screen by projecting means. In this state, the projecting means projects the ophthalmic test image onto either of the deep-bottom hemispherical screen and the shallow-bottom hemispherical screen.

Moreover, the present invention may further include: ophthalmic test type inputting means for inputting the type of the implemented ophthalmic test, and in the present invention, driving means may drive the deep-bottom hemispherical screen to a position, onto which the ophthalmic test image is projected by the projecting means, in a case where the type of the ophthalmic test, which is inputted by the ophthalmic test type inputting means, is a visual field test, and may drive either one of the shallow-bottom hemispherical screen and the deep-bottom hemispherical screen to the position, onto which the ophthalmic test image is projected by the projecting means, in a case where the type of the ophthalmic test, which is inputted by the ophthalmic test type inputting means, is other than the visual field test.

Moreover, in the present invention, the projecting means may include a left eye-ready projection unit and a right eye-ready projection unit, and in a case where the type of the ophthalmic test, which is inputted by the ophthalmic test type inputting means, is one to present separate videos for a left eye and a right eye, the projecting means may perform either of an operation of projecting a stereoscopic video by the left eye-ready projection unit and the right eye-ready projection unit, and an operation of projecting different videos for the left eye and the right eye by the left eye-ready projection unit and the right eye-ready projection unit.

Furthermore, desirably, the present invention further includes: index mark displaying means for displaying an index mark for the visual field test on the deep-bottom hemispherical screen by the projecting means in a case of performing the visual field test.

Furthermore, in the present invention, an entirety of a hemispherical screen with an arbitrary depth may be the deep-bottom hemispherical screen, a bottom portion of the hemispherical screen may be the shallow-bottom hemispherical screen, and a portion of the shallow-bottom hemispherical screen in the hemispherical screen and a portion other than the shallow-bottom hemispherical screen in the hemispherical screen may be composed separately from each other, and the driving means may drive the portion other than the shallow-bottom hemispherical screen in the hemispherical screen, and locate either of the deep-bottom hemispherical screen and the shallow-bottom hemispherical screen at a position onto which the ophthalmic test image is projected by the projecting means.

Furthermore, desirably, the present invention further includes: a planer screen as any of a screen for a vision test, a screen for a binocular vision test, and a screen for an aniseikonia test.

Furthermore, desirably, the present invention further includes: peripheral brightness adjusting means for adjusting brightness of a periphery of either of the deep-bottom hemispherical screen and the shallow-bottom hemispherical screen.

Furthermore, desirably, the present invention further includes: imaging means for imaging an eye portion of a test subject subjected to the ophthalmic test by the ophthalmic test image projected onto either of the deep-bottom hemispherical screen and the shallow-bottom hemispherical screen; and viewpoint position detecting means for detecting a viewpoint position of the test subject by using an eye portion image imaged by the imaging means.

Furthermore, in the present invention, desirably, the imaging means is composed of a plurality of camera devices, each treating the eye portion of the test subject as an imaging range, and the viewpoint position detecting means detects the viewpoint position of the test subject by using a plurality of the eye portion images imaged by the plurality of cameras.

Furthermore, in the present invention, desirably, the imaging means includes: a slide guide portion provided along an outer circumference of the deep-bottom hemispherical screen; and a camera device that moves along the slide guide portion, a plurality of the eye portion images are imaged while moving the camera device along the slide guide portion, and the viewpoint position detecting means detects the viewpoint position of the test subject by using the plurality of eye portion images.

Furthermore, desirably, the present invention further includes: presenting means for presenting, on either of the deep-bottom hemispherical screen and the shallow-bottom hemispherical screen, a mark representing the viewpoint position of the test subject, which is detected by using the eye portion image imaged by the imaging means.

Furthermore, desirably, the present invention further includes: moving means for moving the viewpoint position of the test subject with reference to the viewpoint position detected by the viewpoint position detecting means.

Furthermore, in the present invention, desirably, the viewpoint position detecting means detects a right eye viewpoint position and left eye viewpoint position of the test subject by using the eye portion image imaged by the imaging means, and detects the viewpoint position one more time in a case where a distance between the right eye viewpoint position and the left eye viewpoint position is abnormal.

Furthermore, in the present invention, desirably, the viewpoint position detecting means detects a binocular viewpoint position, right eye viewpoint position and left eye viewpoint position of the test subject by using the eye portion image imaged by the imaging means, and corrects the binocular viewpoint position based on the right eye viewpoint position and the left eye viewpoint position.

### ADVANTAGEOUS EFFECTS OF THE INVENTION

In accordance with the present invention, according to the type of the implemented ophthalmic test, either of the deep-bottom hemispherical screen and the shallow-bottom hemispherical screen is selected, and the ophthalmic test image is projected onto the selected screen. Accordingly, in response to whether or not the visual field, the resolution and the separation are present depending on each of the ophthalmic tests, the various types of ophthalmic tests can be performed by using the screen.

Moreover, in accordance with the present invention, the deep-bottom hemispherical screen is driven to the position, onto which the ophthalmic test image is projected, in the case where the type of the ophthalmic test is the visual field test, and either one of the shallow-bottom hemispherical screen and the deep-bottom hemispherical screen is driven to the position, onto which the ophthalmic test image is projected, in the case where the type of the ophthalmic test is other than the visual field test. Accordingly, a hemispherical screen suitable for each of the ophthalmic tests can be used.

Furthermore, in accordance with the present invention, in the case where the type of the ophthalmic test is one to present the separate videos for the left eye and the right eye, either of the operation of projecting the stereoscopic video and the operation of projecting the different videos for the left eye and the right eye is performed. Accordingly, the ophthalmic test concerned can be performed by using the hemispherical screen.

Furthermore, in accordance with the present invention, the index mark for the visual field test is displayed on the deep-bottom hemispherical screen in the case of performing the visual field test. Accordingly, the test subject is allowed to determine whether or not the index mark can be seen by the visual field thereof, whereby it can be determined how wide the range is, the range being able to be seen by the visual field of the test subject.

Furthermore, in accordance with the present invention, the entirety of the hemispherical screen with the arbitrary depth is the deep-bottom hemispherical screen, and the bottom portion of the hemispherical screen is formed as the shallow-bottom hemispherical screen. Accordingly, the various types of ophthalmic tests can be performed by using the hemispherical screens different in depth from each other, which are made of the single hemispherical screen.

Furthermore, in accordance with the present invention, the planer screen is provided as any of the screen for the vision test, the screen for the binocular vision test and the screen for the aniseikonia test. Accordingly, the ophthalmic test using the planer screen can be performed in addition to the ophthalmic tests using the hemispherical screen.

Furthermore, in accordance with the present invention, the brightness of the periphery of either of the deep-bottom hemispherical screen and the shallow-bottom hemispherical screen is adjusted. Accordingly, the various types of ophthalmic tests can be realized in a circumstance where the brightness is adjusted.

Furthermore, in accordance with the present invention, the eye portion of the test subject subjected to the ophthalmic test by the ophthalmic test image projected onto either of the deep-bottom hemispherical screen and the shallow-bottom hemispherical screen is imaged, and the viewpoint position of the test subject is detected. Accordingly, it is unnecessary for a third party to confirm the viewpoint position of the test subject, and an environment can be realized, where the test subject can concentrate on the ophthalmic test.

Furthermore, in accordance with the present invention, the viewpoint position of the test subject is detected by using the plurality of eye portion images imaged by the plurality of camera devices. Accordingly, a more accurate viewpoint position can be detected.

Furthermore, in accordance with the present invention, the plurality of eye portion images are imaged while moving the camera device along the slide guide portion, and the viewpoint position of the test subject is detected by using the plurality of eye portion positions. Accordingly, a more accurate viewpoint position can be detected, and in addition, cost reduction of the device, and the like can be realized.

Furthermore, in accordance with the present invention, the mark representing the viewpoint position of the test subject is presented on either of the deep-bottom hemispherical screen and the shallow-bottom hemispherical screen. Accordingly, the test subject and the third party can be notified of the viewpoint position, and the viewpoint position can be moved to a desirable viewpoint position.

Furthermore, in accordance with the present invention, the moving means for moving the viewpoint position of the test subject with reference to the detected viewpoint position is provided. Accordingly, the viewpoint position is moved to a viewpoint position desirable for the ophthalmic test, whereby the test subject can be subjected to the ophthalmic test in an appropriate state.

Furthermore, in accordance with the present invention, in the case where the distance between the right eye viewpoint position and the left eye viewpoint position is abnormal, the viewpoint position is detected one more time. Accordingly, the viewpoint position of the test subject can be detected by using an image imaged in a normal state, and the ophthalmic test can be performed.

Furthermore, in accordance with the present invention, the binocular viewpoint position, right eye viewpoint position and left eye viewpoint position of the test subject are detected, and the binocular viewpoint position is corrected based on the right eye viewpoint position and the left eye viewpoint position. Accordingly, an accurate binocular viewpoint position can be obtained from the right eye viewpoint position and the left eye viewpoint position.

### BRIEF DESCRIPTION OF THE DRAWINGS

FIG. 1 is a perspective view of a multifunctional ophthalmic test device shown as an embodiment of the present invention.
FIG. 2 is a perspective view showing a state of using a vision test screen in place of a hemispherical screen in the multifunctional ophthalmic test device shown as the embodiment of the present invention.
FIG. 3(a) is a view showing a state where the hemispherical screen in the multifunctional ophthalmic test device shown as the embodiment of the present invention is composed as a deep-bottom hemispherical screen, and FIG. 3(b) is a view showing a state where the hemispherical screen is composed as a shallow-bottom hemispherical screen.
FIG. 4 is a block diagram showing a functional configuration of the multifunctional ophthalmic test device shown as the embodiment of the present invention.
FIG. 5 is a flowchart showing operations of the multifunctional ophthalmic test device shown as the embodiment of the present invention.
FIG. 6 is a table showing an example of ophthalmic tests capable of being implemented by the multifunctional ophthalmic test device shown as the embodiment of the present invention.
FIG. 7(A) is a schematic view where a camera device takes, as an imaging range, a region including eyes of a test subject who is putting the chin on a viewpoint fixing mechanism, FIG. 7(B) is an eye portion image acquired by the camera device, FIG. 7(C) is a schematic view where a viewpoint position of the test subject is detected from an image detected by a corneal reflex of the eyes by performing infrared radiation therefor, and FIG. 7(D) is an eye portion image acquired by the camera device and including eyeball images and corneal reflex images.
FIG. 8 is a perspective view showing a state where a mark representing a viewpoint position is displayed on the hemispherical screen in the multifunctional ophthalmic test device shown as the embodiment of the present invention.
FIG. 9 is a perspective view showing a configuration in which the camera device is movable along an outer circumference of the hemispherical screen in the multifunctional ophthalmic test device shown as the embodiment of the present invention.

### BEST MODE FOR CARRYING OUT THE INVENTION

A description is made below of an embodiment of the present invention with reference to the drawings.

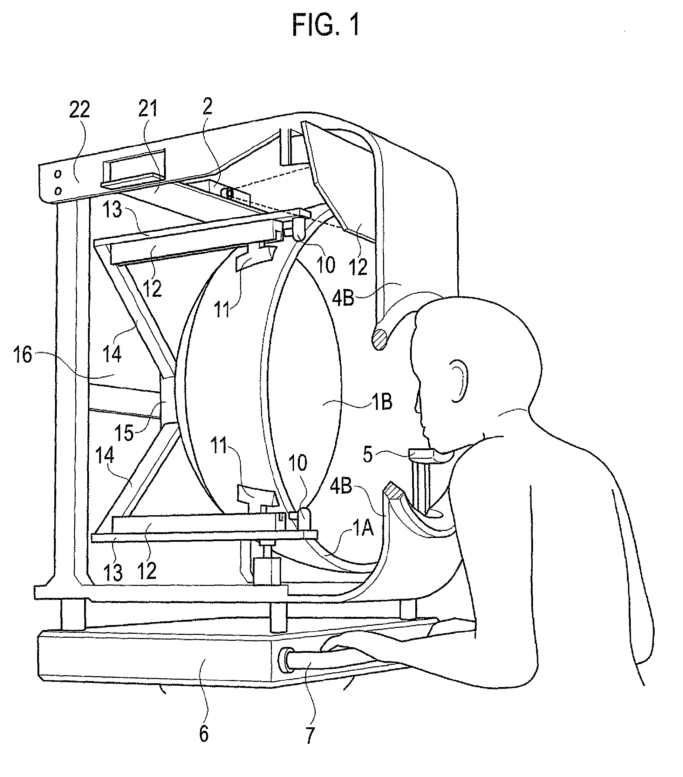

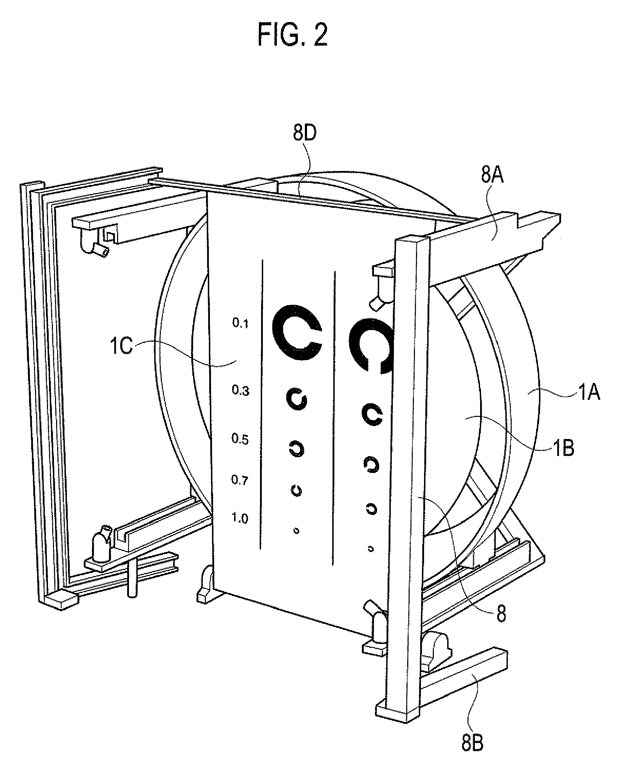

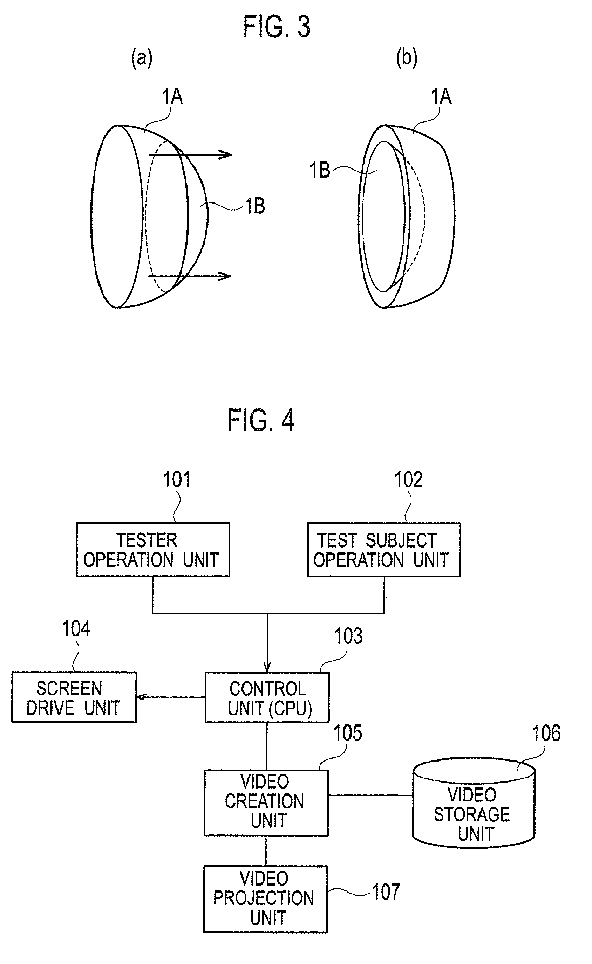

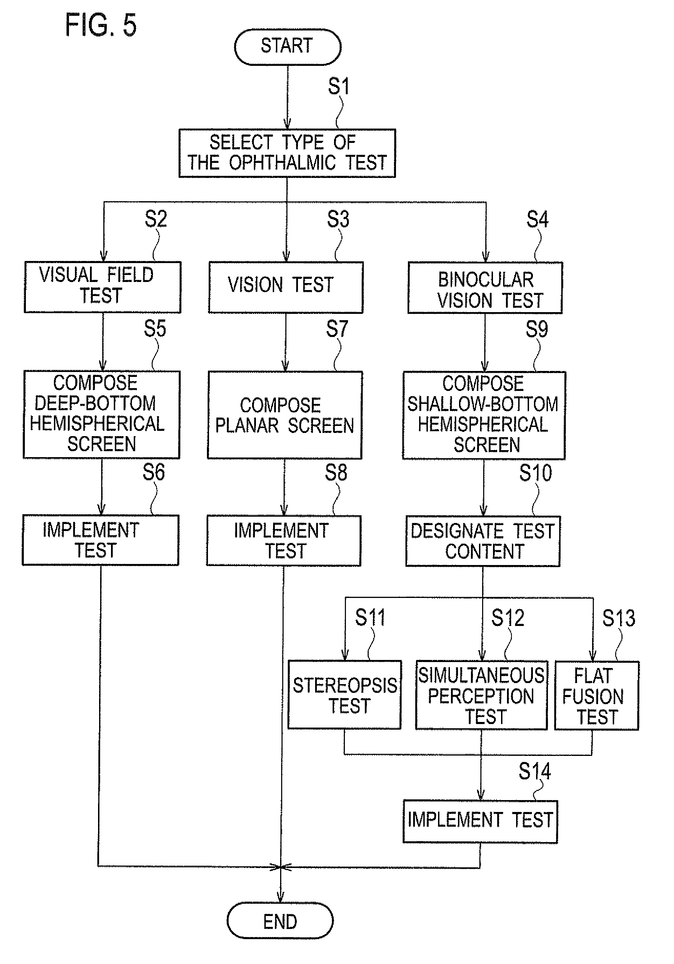

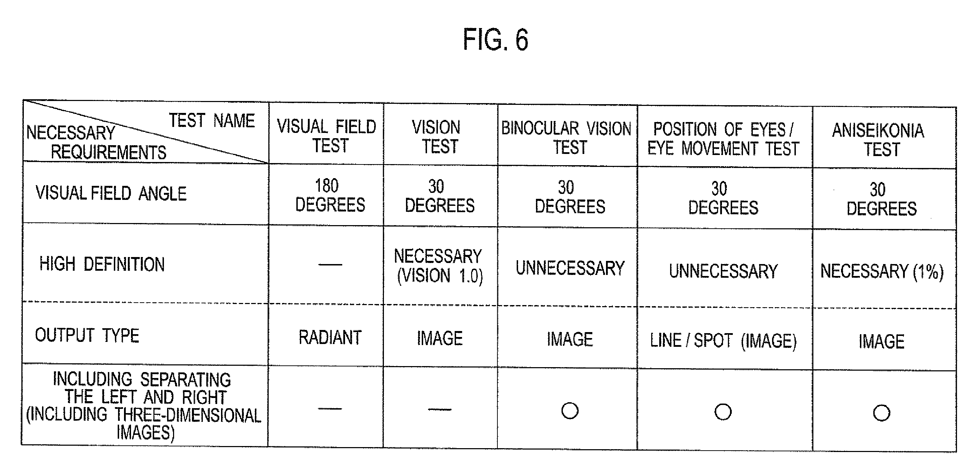

A multifunctional ophthalmic test device shown as the embodiment of the present invention is configured, for example, as shown in FIG. 1. FIG. 1 shows a perspective view of the multifunctional ophthalmic test device. FIG. 2 shows a perspective view where a planar screen is attached to the multifunctional ophthalmic test device. FIG. 3 shows a state transition of a hemispherical screen. FIG. 4 shows a functional configuration of the multifunctional ophthalmic test device. FIG. 5 shows operations of the multifunctional ophthalmic test device. FIG. 6 shows a table where operation states of the multifunctional ophthalmic test device are summarized.

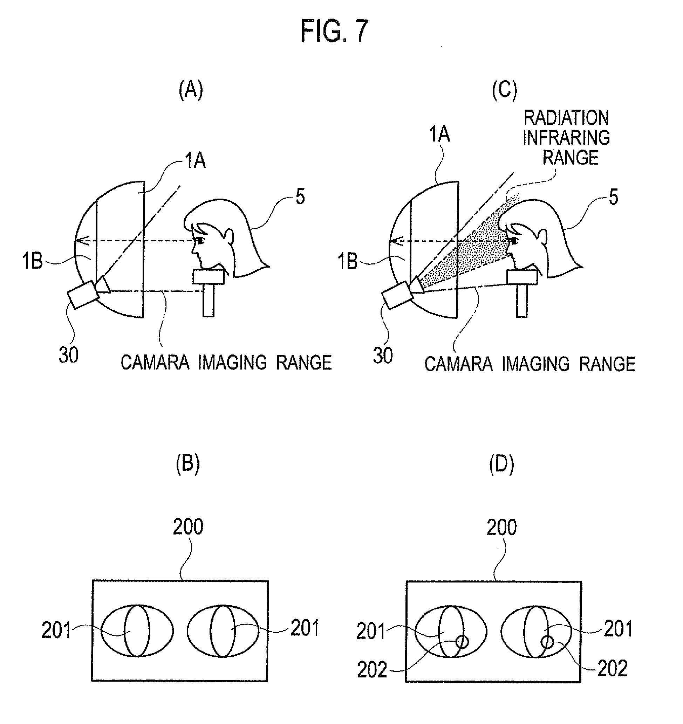

As shown in FIG. 1, this multifunctional ophthalmic test device is a device, which allows a face of a test subject to be located so as to directly confront a hemispherical screen 1 (also called a dome-type screen), and performs a plurality of ophthalmic tests for the test subject concerned. Note that, in this embodiment, a description is made of a configuration of projecting a video on the "hemispherical screen"; however, the screen is not limited to a hemispherical shape. Specifically, the screen does not need to be "hemispherical", or even to be a "half of a sphere", and the matter that the screen is a "dome-type" screen as in FIG. 3 and FIG. 7 is only referred to as "semispherical". Hence, the invention of this application, which is described below, is also applicable to the "dome-type screen).

This multifunctional ophthalmic test device can perform at least a "visual field test" and a "binocular vision test", which are shown in FIG. 6 and are frequently performed in ophthalmologists. As shown in FIG. 1, though adopting the hemispherical screen 1 generally used in the visual field test, this multifunctional ophthalmic test device can implement other ophthalmic tests such as a vision test. Moreover, as the other ophthalmic tests which can be performed by this multifunctional ophthalmic test device, there are mentioned a "binocular visual function test (simultaneous perception/flat fusion/stereopsis)", a "position of eyes/"eye movement test", and an "aniseikonia test". A description is made below of such a multifunctional ophthalmic test device.

### [Configuration of multifunctional ophthalmic test device]

The multifunctional ophthalmic test device shown in FIG. 1 includes: the hemispherical screen 1; a projector 2 as projecting means for emitting video light projected onto the hemispherical screen 1 concerned; a mirror 3 that reflects the video light, which is emitted from the projector 2 concerned, toward the hemispherical screen 1; and a viewpoint fixing mechanism 5 that sets a face position of the test subject at a predetermined position with respect to the hemispherical screen 1. Moreover, this multifunctional ophthalmic test device also includes a configuration, in which a unit having the hemispherical screen 1, the projector 2, the mirror 3 and the viewpoint fixing mechanism 5 as main constituents is mounted on a mounting pedestal 6, and a grab rail 7 provided on the mounting pedestal 6 concerned is gripped by the test subject during the test.

In the hemispherical screen 1, the entirety of the hemispherical screen 1 with an arbitrary depth is formed as a deep-bottom hemispherical screen, and a bottom portion thereof with a recessed shape, which includes a center portion of the hemispherical screen 1, is formed as a shallow-bottom hemispherical screen 1B. That is to say, in the hemispherical screen 1 concerned, a portion of the shallow-bottom hemispherical screen 1B and a portion other than the shallow-bottom hemispherical screen 1B of the hemispherical screen 1 concerned are composed separately from each other. Note that, in the following description, the entirety of the hemispherical screen 1 shown in FIG. 1 is referred to as a deep-bottom hemispherical screen 1A, and a screen portion including the bottom portion in the entirety of the hemispherical screen 1 is referred to as a shallow-bottom hemispherical screen 1B.

Note that, though the screen that projects the video thereon in this embodiment is referred to as a "hemispherical screen", the screen does not always need to be hemispherical, and just needs to have such a shape as composing a part of a sphere like the deep-bottom and shallow-bottom screen as shown in FIG. 1.

The multifunctional ophthalmic test device as described above can be switched between a state as shown in FIG. 3(a), where the deep-bottom hemispherical screen 1A is composed of the shallow-bottom hemispherical screen 1B and a peripheral edge portion, which compose the hemispherical screen 1 concerned, and a state as shown in FIG. 3(b), where the hemispherical screen 1 is composed only of the shallow-bottom hemispherical screen 1B. Note that, in the case where the transition is made from the state of FIG. 3(a) to the state of FIG. 3(b), not only the case where the peripheral edge portion of the hemispherical screen 1 moves backward may be allowed, but also a configuration in which the shallow-bottom hemispherical screen 1B moves forward may be adopted. Moreover, in the case where a hemispherical screen 1 formed of a easily deformable member such as cloth and of wires which support the same is adopted, the screen can also be deformed by changing positions of the wires.

Returning to FIG. 1, the multifunctional ophthalmic test device includes; four screen support portions 11 which support four ends of the peripheral edge portion that composes the hemispherical screen 1; and four slide guide portions 12 which enable the respective screen support portions 11 to slide thereon. The hemispherical screen 1 is supported by arms 13 and 14, a fixing member 15 and a back surface portion 16, which support the peripheral edge portion and the shallow-bottom hemispherical screen 1B, while interposing the screen support portions 11 and the slide guide portions 12 therebetween. In the multifunctional ophthalmic test device as described above, on the peripheral edge portion of the hemispherical screen 1, the screen support portions 11 are driven along the slide guide portions 12 by drive motors (not shown). In such a way, the peripheral edge portion of the hemispherical screen 1 is driven in a fore-and-aft direction with respect to the test subject, and makes the state transition between FIG. 3(a) and FIG. 3(b).

The projector 2 is provided on an installation pedestal 21 provided on a pair of arms 22 connected to a substantial upper end portion of the back surface portion 16 of the hemispherical screen 1. The pair of arms 22 are extended from the back surface portion 16 to the test subject side, and the mirror is attached to tip end portions of the arms 22 concerned. In such a configuration, the multifunctional ophthalmic test device is designed so that a relationship among a position of the projector 2 on the installation pedestal 21, a position of the mirror 3, which is determined by the arms 22, and a posture of the mirror 3 with respect to the projector 2 and the hemispherical screen 1 can be an appropriate relationship. That is to say, a projection range of the projector 2 is changed, whereby switching is made possible between a state where the video can be projected onto the entirety of the deep-bottom hemispherical screen 1A, which includes the entirety of the hemispherical screen 1, and a state where the video can be projected only onto the shallow-bottom hemispherical screen 1B.

As necessary requirements corresponding to the variety of tests are shown in FIG. 6, the multifunctional ophthalmic test device configured as described above is required to have a visual field as wide as 180 degrees at the time of the visual field test, and it is sufficient if a visual field as 30 degrees can be ensured in the ophthalmic tests other than the visual field test. Hence, in the multifunctional ophthalmic test device, at the time of the visual field test, the hemispherical screen 1 is composed as the deep-bottom hemispherical screen 1A as shown in FIG. 3(a). Meanwhile, at the time of the other ophthalmic tests, the hemispherical screen 1 is composed of the shallow-bottom hemispherical screen 1B as shown in FIG. 3(b).

Note that, in this embodiment, the example is shown, where the hemispherical screen 1 is divided into two pieces which are the shallow-bottom hemispherical, screen 1B and the peripheral edge portion of the hemispherical screen 1; however, the hemispherical screen 1 is not limited to this. Specifically, in this embodiment, as shown in FIG. 6, only the case is shown, where there are two types of the visual field, which are 180 degrees and 30 degrees. However, without being limited to this, it is desirable that the visual field be switched in plural stages, which are two or more, so that an angular range optimal for performing the variety of ophthalmic tests or an angle corresponding to a preference of the test subject can be realized. For this purpose, it is desirable that the hemispherical screen 1 can be divided not only into two pieces but also into pieces with a division number that is two or more. For example, in the case where the shallow-bottom hemispherical screen 1B is formed as the one that realizes the visual field of 30 degrees, the peripheral edge portion of the hemispherical screen 1 is further divided into portions which individually realize a visual field of 60 degrees, 90 degrees, and so on. Then, it is desirable that peripheral edge portions of the hemispherical screen 1, which are thus divided, be moved back and forth by motors corresponding thereto individually.

Moreover, angles of screen surfaces in the deep-bottom hemispherical screen 1A and the shallow-bottom hemispherical screen 1B, when seen from a center portion of an opening surface therein, are not limited to 180 degrees and 30 degrees, respectively. For example, the angle in the deep-bottom hemispherical screen 1A may be 210 degrees or less, and the angle in the shallow-bottom hemispherical screen 1B may be 30 degrees or more.

Furthermore, in this multifunctional ophthalmic test device, at the time of the vision test, as shown in FIG. 2, a vision test screen 1C that is a planer screen as a vision test table may be used in place of the shallow-bottom hemispherical screen 1B in the configuration shown in FIG. 1. For this vision test screen 1C, the one on which Landolt rings are drawn is used.

This vision test screen 1C is attached to a screen housing portion 8D provided between pairs of arms 8, 8A and 8B provided outside of a support mechanism of the hemispherical screen 1, which is composed of the arms 13 and 14 and the fixing member 15. In this screen housing portion 8D, motors for taking up/drawing out the vision test screen 1C are connected to both ends thereof to which the arms 8A are connected. Then, at the time of using the vision test screen 1C, the screen housing portion 8D is driven in a direction where the peripheral edge portion of the hemispherical screen 1 is spaced apart from the test subject, and is then driven in a direction of drawing out the vision test screen 1C. As opposed to this, in the case of not using the vision test screen 1C, the vision test screen 1C is taken up by the screen housing portion 8D, whereby it becomes possible to perform the ophthalmic tests by the deep-bottom hemispherical screen 1A or the shallow-bottom hemispherical screen 1B.

Here, in the case of performing the vision test, no matter which of the vision test screen 1C and the shallow-bottom hemispherical screen 1B may be used, it is desirable to adjust brightness on the periphery of the vision test screen 1C or the shallow-bottom hemispherical screen 1B. Here, as a configuration of adjusting the brightness of the periphery, there is mentioned a configuration of adjusting illuminance of a lamp in a room, in which the multifunctional ophthalmic test device is provided, in conjunction with the operations of the multifunctional ophthalmic test device.

Moreover, without being limited to the vision test, in the case of desiring to keep a region on the hemispherical screen 1 at constant brightness, an ND filter matched with brightness of the video and the shape of the hemispherical screen 1 can be designed and used. Furthermore, in the multifunctional ophthalmic test device, in the case of keeping a region, which connects the hemispherical screen 1 and the viewpoint position of the test subject to each other, at even brightness, then illumination and a measuring instrument such as a light meter (illuminometer) are arranged in the multifunctional ophthalmic test device, whereby the brightness can always be kept even.

Note that the planer screen such as the vision test screen 1C may perform not only the vision test but also the aniseikonia test by drawing a test image for the aniseikonia thereon. With regard to such tests using the planer screen as described above, a variety of planer screens are prepared by a tester in advance, whereby the plurality of ophthalmic tests are realized by using the planer screens more easily.

Moreover, the planer screens as described above may be used as the vision test screen, a binocular vision test screen and an aniseikonia test screen. The planer screens may be those on which picture patterns corresponding to various types of test contents are drawn, or may be those which display videos thereon.

As shown in FIG. 4, in terms of a functional configuration, the multifunctional ophthalmic test device configured as described above includes: tester operation unit 101 operated by the tester of the ophthalmic tests; a test subject operation unit 102 operated by the test subject; a control unit 103 composed of a CPU and the like; a screen drive unit (driving means) 104 composed of motors and the like, which drive the above-mentioned screen support portions 11 along the slide guide portions 12; a video creation unit 105 that creates video data for generating video light projected onto the screen; a video storage unit 106 that stores the video data for implementing the various types of ophthalmic tests; and a video projection unit 107 that corresponds to the above-mentioned projector 2.

In this multifunctional ophthalmic test device, at the time of implementing the ophthalmic tests, the tester operation unit 101 is operated, and information regarding which type of the ophthalmic tests is to be implemented is inputted (ophthalmic test type inputting means). Though not shown, the tester operation unit 101 may be an operation unit provided on any region of the multifunctional ophthalmic test device, or may be a keyboard and mouse of a personal computer separate from the multifunctional ophthalmic test device. The type of the ophthalmic test, which is created by this tester operation unit 101, is supplied to the control unit 103.

When the video light for implementing the ophthalmic test is projected onto the hemispherical screen 1, the test subject operation unit 102 is operated in response to a state where the test subject recognizes how the video concerned looks. This test subject operation unit 102 may be a button-type or lever-type operation unit operated directly by the test subject. However, in the case where the test subject vocally tells the tester how the video looks, an operation unit for which the tester performs an input operation in response to voice thus transmitted serves as the test subject operation unit 102. Operation signals indicating such an operation for the test subject operation unit 102 are supplied to the control unit 103.

The control unit 103 determines the type of the implemented ophthalmic test based on the operation signals from the tester operation unit 101 and the test subject operation unit 102. Then, based on the type of the implemented ophthalmic test, the control unit 103 determines which of the ophthalmic test using the deep-bottom hemispherical screen 1A and the ophthalmic test using the shallow-bottom hemispherical screen 1B is to be performed. Based on a result of this determination, the control unit 103 controls the screen drive unit 104, and makes transition for the state of the hemispherical screen 1 to either state of the deep-bottom hemispherical screen 1A and the shallow-bottom hemispherical screen 1B. As mentioned above, in the case where the designated type of the ophthalmic test is the "visual field test", the deep-bottom hemispherical screen 1A is used, and in the case where the designated type of the ophthalmic test is "other than the visual field test", the shallow-bottom hemispherical screen 1B is used. Note that, in this embodiment, the example is shown, where the shallow-bottom hemispherical screen 1B is used for the ophthalmic tests other than the visual field test. However, without being limited to this, the ophthalmic tests other than the visual field test may naturally be performed by either of the shallow-bottom hemispherical screen 1B or the deep-bottom hemispherical screen 1A. Moreover, as examples of using the deep-bottom hemispherical screen 1A for the ophthalmic tests other than the visual field test, a deep-bottom hemispherical screen 1A that can ensure the visual field of 180 degrees also in the binocular visual function test, a dynamic visual field test and the aniseikonia test may be used in examples other than the example shown in FIG. 6.

As this visual field test, there are: a visual field test using a video; and a visual field test using index mark displaying means such as a pointer. The visual field test using the video refers to a test performed by presenting a target (light spot) for the visual field test by projecting a video having only one lighting point onto a screen. Moreover, the visual field test using the index mark displaying means refers to display of an index mark for the visual field test on the deep-bottom hemispherical screen 1A by target presentation mechanisms 10. As this index mark displaying means, there is mentioned a pointer that displays the spot on the hemispherical screen 1 by being operated by the tester. This pointer builds a fine light source in a tip end portion thereof, and is configured so that an orientation thereof can be changed toward an inner surface of the hemispherical screen 1 and the index mark can be outputted therefrom at the time when the index mark is displayed by the pointer concerned. This index mark for the visual field test is a light spot that enables the test subject to determine whether or not the light spot concerned can be seen in the visual field thereof. Then, in accordance with a manual operation for the pointer by the tester, the control unit 103 increases and reduces a range of the light spot on the hemispherical screen 1, and can thereby determine which range the test subject can see by the visual field thereof.

Moreover, based on the designated type of the ophthalmic test, the control unit 103 controls the video light projected from the video light projection unit 107 (projector 2) through the mirror 3 onto the hemispherical screen 1. In such a way, in accordance with the control of the control unit 103, the video creation unit 105 reads out video data of a type corresponding to the designated type of the ophthalmic test from the video storage unit 106, and supplies the video data to the video projection unit 107. As shown in FIG. 6, in the case where the designated type of the ophthalmic test is the "visual field test", video data for projecting the light spot onto the deep-bottom hemispherical screen 1A is read out. In the case where the designated type of the ophthalmic test is the "vision test", the "binocular visual function test" or the "aniseikonia test", video data indicating a video is read out. In the case where the designated type of the ophthalmic test is the "position of eyes/eye movement test", video data of a video composed of lines and points is read out.

Furthermore, it is necessary that the control unit 103 satisfy resolution required, based on the designated type of the ophthalmic test, for the video projected onto the hemispherical screen 1. As shown in FIG. 6, particularly in the case where the designated type of the ophthalmic test is the "vision test", the video is required to have high resolution equivalent to 20/20 vision. Moreover, in the case where the designated type of the ophthalmic test is the "aniseikonia test", the video is required to have resolution to an extent where a difference of 1% is distinguishable since the test subject sees an object video on the hemispherical screen 1. It is desirable that this resolution be set in advance in the video data itself, which is stored in the video storage unit 106, in response to the types of the ophthalmic tests. However, in the case where the resolution corresponding to each type of the ophthalmic tests cannot be realized depending on the video data concerned, it is necessary that the control unit 103 control the video projection unit 107 to realize the required resolution.

Moreover, in the multifunctional ophthalmic test device, in the case of performing the binocular visual function test, it is desirable that the video projection unit 107 be composed of two projectors 2. Specifically, the video projection unit 107 is configured to have a left eye-ready projection unit and a right eye-ready projection unit. Then, in the case where the type of the ophthalmic test, which is inputted by the tester operation unit 101, is the binocular vision test, the control unit 103 performs an operation of projecting a stereoscopic video by the left eye-ready projection unit concerned and the right eye-ready projection unit concerned. In this case, for example, a polarization filter for the left eye is provided in the left eye-ready projection unit, and a polarization filter for the right eye is provided in the right eye-ready projection unit. Then, a left eye portion of polarization glasses worn by the test subject is formed as a polarization filter for the left eye, and a right eye portion thereof is formed as a polarization filter for the right eye.

Furthermore, in the case of performing the position of eyes/eye movement test, the simultaneous perception test, and the aniseikonia test, which allow the right eye and the left eye to visually recognize different images, the multifunctional ophthalmic test device performs an operation of projecting video light as allowing the left eye and the right eye to visually recognize the different videos by the left eye-ready projection unit and the right eye-ready projection unit.

A description of the operations of the above-described multifunctional ophthalmic test device is made with reference to FIG. 5. First, which type of the ophthalmic test is to be performed is selected by the operation of the tester for the test subject operation unit 102 (Step S1). Then, for example, which of the visual field test (Step S2), the vision test (Step S3) and the binocular vision test (Step S4) is to be performed is determined by the control unit 103. Note that it is known that there are approximately 18 types as the ophthalmic tests, and naturally, the ophthalmic tests are not limited to the examples shown in FIG. 5 and FIG. 6.

Then, when it is determined in Step S2 that the visual field test is to be performed, the screen drive unit 104 is controlled by the control unit 103 to compose the deep-bottom hemispherical screen 1A (Step S5), and the visual field test is implemented (Step S6).

In this visual field test of Step S6, the deep-bottom hemispherical screen 1A is used, and the light spot is moved in a desired direction to be seen, the light spot being outputted from the video projection unit 107 by the control unit 103, and being projected onto the deep-bottom hemispherical screen 1A. Then, the test subject is allowed to perform an input operation for the test subject operation unit 102 at the time of sensing the light spot without moving the eye, whereby it is recognized how wide the visual field of the test subject is. Moreover, this visual field test is repeatedly performed while changing a light quantity and size of the light spot, and a result of the visual field test concerned is recorded in a storage unit (not shown).

When it is determined in Step S3 that the vision test is to be performed, the screen drive unit 104 is controlled by the control unit 103 to present the vision test screen 1C in front of the test subject (Step S7), and the vision test is implemented (Step S8). Here, the control unit 103 controls the screen drive unit 104 to drive the peripheral edge portion of the hemispherical screen 1 backward, and in addition, performs controls to drive the motors for taking up/drawing out the vision test screen 1C, and to thereby draw out the vision test screen 1C.

In this vision test of Step S8, the vision test screen 1C is used, the test subject is allowed to input, to the test subject operation unit 102, a recognition state of the Landolt rings drawn on the vision test screen 1C concerned, and a result of the visual test concerned is recorded in the storage unit (not shown). Here, in order to accurately perform the vision test, the brightness on the periphery of the vision test screen 1C or the shallow-bottom hemispherical screen 1B is adjusted no matter which of the vision test screen 1C and the shallow-bottom hemispherical screen 1B may be used. For example, desirably, the illuminance of the room lamp is adjusted before implementing the vision test, whereby the index mark is shown at constant brightness from the front surface and back surface of the vision test screen 1C, and in addition, the room lamp is automatically adjusted at illuminance at which the tester determines to perform the vision test. In such a way, a test of night vision or the like can be realized. Moreover, a test of dynamic vision or the like can also be realized by using the video.

Note that, naturally, the vision test may be performed by using the shallow-bottom hemispherical screen 1B with a visual field angle of approximately 30 degrees without using the vision test screen 1C. In this case, the control unit 103 controls the video creation unit 105 and the video projection unit 107 to display the index mark on the shallow-bottom hemispherical screen 1B.

When it is determined in Step S4 that the binocular vision test is to be performed, the screen drive unit 104 is controlled by the control unit 103 to present the shallow-bottom hemispherical screen 1B in front of the test subject (Step S9). Here, the control unit 103 controls the screen drive unit 104 to drive the peripheral edge portion of the hemispherical screen 1 backward, and thereby makes a state where the shallow-bottom hemispherical screen 1B is usable. Moreover, the control unit 103 recognizes a test content designated in the binocular vision test based on an operation for the tester operation unit 101 (Step S10).

By the operation of the tester for the tester operation unit 101, which of a stereopsis test (Step S11), a simultaneous perception test (Step S12) and a flat fusion test (Step S13) in the binocular visual function test is to be performed is selected, and the selected test is implemented (Step S14).

In the stereopsis test, the shallow-bottom hemispherical screen 1B is used, an index mark provided with a parallax is presented so as to correspond to the left eye and the right eye, and based on the operation for the test subject operation unit 102, the tester is allowed to confirm whether or not the test subject stereoscopically senses the index mark concerned. For example, the stereopsis test is performed by a test, in which index marks provided with the parallax and index marks provided with no parallax are presented, and the test subject is allowed to answer stereoscopic ones among a plurality of the index marks by the operation for the test subject operation unit 102.

In the simultaneous perception test, the shallow-bottom hemispherical screen 1B is used, the left eye and the right eye are allowed to visually recognize different images, and the test subject is allowed to operate the test subject operation unit 102 so as to superpose the left eye video and the right eye video on each other. For example, the multifunctional ophthalmic test device presents a picture pattern of a lion as one of videos, and presents a video of a cage as the other one of the videos. Then, the multifunctional ophthalmic test device allows the test subject to operate the test subject operation unit 102 so as to put the lion into the cage, and records, as a test result of the simultaneous perception test, a positional shift between the picture pattern of the lion and the picture pattern of the cage in a state where the test subject perceives that the lion is put into the cage. In such a way, the multifunctional ophthalmic test device can test a positional shift between the right and left eyes and a recognition shift therebetween.

The flat fusion test is a test that allows the test subject to operate the test subject operation unit 102 at the point of time when the test subject can perceive that there are two videos which are a left eye video and a right eye video as a result that the left eye video and the right eye video are gradually separated from each other from a state where a single video is displayed on the shallow-bottom hemispherical screen 1B so that the left eye video and the right eye video can be perceived like being superposed on each other . Then, the multifunctional ophthalmic test device records a movement angle between the left eye video and the right eye video at the point of time when the test subject operation unit 102 is operated as a limit of fusing the left eye video and the right eye video into one. In such a way, the multifunctional ophthalmic test device investigates to which point the two videos can be perceived as one by allowing the test subject to do cross eye and wall eye.

Note that the multifunctional ophthalmic test device may implement the position of eyes/eye movement test and the aniseikonia test in addition to the binocular vision test and the simultaneous perception test.

As described above in detail, in accordance with the multifunctional ophthalmic test device shown as the present invention, the deep-bottom hemispherical screen 1A or the shallow-bottom hemispherical screen 1B can be used as the hemispherical screen 1, and accordingly, the ophthalmic tests with the number of types, which cannot be achieved by a single device heretofore, can be performed. Specifically, as shown in FIG. 6, in response to whether or not the visual field, the resolution and the separation are present depending on each of the ophthalmic tests, the switching of the screen, the switching of the resolution, and the image separation are performed, whereby the various types of ophthalmic tests can be performed. Hence, in accordance with this multifunctional ophthalmic test device, it is not necessary to install instruments dedicated individually to many types of the ophthalmic tests, and a space for the dedicated instruments concerned becomes unnecessary, and it also becomes unnecessary to move the test subject.

Note that the above-mentioned embodiment is an example of the present invention. Therefore, the present invention is not limited to the above-mentioned embodiment, and naturally, it is possible to make a variety of changes in response to the design and the like within the scope without departing from the technical idea according to the present invention even in other than this embodiment.

Specifically, in the visual field test using the above-mentioned deep-bottom hemispherical screen 1A, an illumination color of a background of the light spot projected onto the deep-bottom hemispherical screen 1A can also be changed, and a color of the light spot can also be changed. Moreover, in the multifunctional ophthalmic test device, in order to confirm that the test subject does not move the eye, a fixation point monitoring camera may be provided, or a position of a pupil can be automatically determined by tracking the same to check whether or not the test subject moves the eye. Furthermore, in the case where the test subject moves the eye, the multifunctional ophthalmic test device may issue a notice of this matter by voice, and may automatically eliminate a result in this case from the test results.

Moreover, in the vision test using the above-mentioned shallow-bottom hemispherical screen 1B or vision test screen 1C, a lens and a mirror may be arranged between the index mark and the test subject, and the index mark may be thereby shown like being located at a designated distance, a contrast and brightness may be changed by using the lens, a filter and the like, or only a portion of the index mark, which is desired to be shown, may be brightened by the lens, the filter, a diaphragm and the like. Moreover, in order to select the index mark for the vision test, the designated index mark may be lighted by an operation for a button or the like, which composes the tester operation unit 101, the vision test may be performed by displaying the index mark based on a content programmed in advance, or the vision test may be performed by automatic determination based on the number of true/false answers made in a designation order.

Furthermore, in the binocular visual function test using the shallow-bottom hemispherical screen 1B, a size, position and color tone of the stereoscopic video may be changed based on the designated content, or a test result of the binocular visual function test may be made capable of being outputted and recorded in a preferred unit such as a visual angle. Moreover, commonly to the position of eyes/eye movement test and the aniseikonia test, for a dichoptic method between the left eye video and the right eye video, an anaglyph (red/green glasses) mode and a spectral mode can be used, which can realize the tests by using one projection unit and not by two units which are the left eye-ready projection unit and the right eye-ready projection unit. Moreover, desirably, a stereoscopic video conversion unit is provided in the video creation unit 105, thus enabling data conversion in accordance with each of the spectral mode and the polarization mode.

Still further, a configuration may be adopted so that, in the position of eyes/eye movement test, a motion can be electrically inputted with respect to an operation for the operation unit, which is of moving an arrow of a mouse, a touch panel or the like, and that the inputted motion can be outputted and recorded.

Still further, in the aniseikonia test, in accordance with the operation of the tester for the tester operation unit 101, the index mark may be presented while changing the size thereof to a predetermined width (1%, 100 seconds and the like), or the size of the index mark may be dynamically changed by using, as the tester operation unit 101, an operation mechanism such as a joystick and a knob.

Still further, desirably, this multifunctional ophthalmic test device can receive and manage test subject information regarding the ophthalmic test, a result of each ophthalmic test and the like, and desirably, the multifunctional ophthalmic test device can make a database of the results of such ophthalmic tests, and compare the results concerned with those collected at the previous time and those of other persons. Moreover, the region to which the video is presented may be switched in the hemispherical screen 1, the optical system of the projector 2 may be changed, or the video may be projected only onto a part of the hemispherical screen 1. Furthermore, at the time of presenting the index mark or the parallax as a video required to be fine and accurate, a portion onto which the video is projected may be changed in such a manner that only a part of the hemispherical screen 1 is used.

Next, in the multifunctional ophthalmic test device shown as the embodiment of the present invention, a description is made of the one capable of performing the ophthalmic tests while detecting an accurate viewpoint position of the test subject by monitoring an eye state of the test subject.

Before describing a specific configuration of the multifunctional ophthalmic test device, a description is made of a significance of confirming the viewpoint position of the test subject by the multifunctional ophthalmic test device shown as the embodiment of the present invention.

In the above-mentioned multifunctional ophthalmic test device, at the time of performing the various types of ophthalmic tests shown in FIG. 6, it becomes an important point to grasp the viewpoint position as to where on the hemispherical screen 1 the test subject is seeing, and it is necessary to perform the ophthalmic tests while confirming whether the viewpoint position of the test subject is present within an appropriate range.

Therefore, in the conventional ophthalmic test instrument, there have been adopted a method of visually confirming the viewpoint position of the test device by an optometrist, and a method of moving the viewpoint position in such a manner that the test subject him/herself adjusts a chin support and the like of the viewpoint fixing mechanism 5 to a target position for performing the ophthalmic tests. Moreover, as the ophthalmic care has developed, ophthalmic tests in a state more approximate to the daily life (that is, also called a daily vision) have been required. In the case of performing the ophthalmic tests in the daily vision, unlike the conventional ophthalmic tests, it sometimes becomes necessary to obtain the test results by grasping to which direction the test subject is facing without fixing the viewpoint position.

In this connection, means for detecting the viewpoint position is provided in the above-mentioned multifunctional ophthalmic device, thus making it possible to confirm the viewpoint position from the optometrist and the test subject him/herself, or making it possible to record and confirm the viewpoint position of the ophthalmic test results.

Specifically, as shown in FIG. 7, a camera device 30 (imaging means) is provided, which images an eye portion of the test subject subjected to each of the ophthalmic tests by an ophthalmic test image projected onto the deep-bottom hemispherical screen 1A or the shallow-bottom hemispherical screen 1B. Then, the multifunctional ophthalmic test device detects the viewpoint position of the test subject by the CPU including the control unit 103 by using an eye portion image imaged by the camera device 30 (viewpoint position detecting means). A notice on such a viewpoint position of the test subject, which is detected by the CPU, is issued to the optometrist and the test subject him/herself. In such a way, the ophthalmic test technician and the test subject can get to know whether or not the viewpoint position is optimal for the ophthalmic test.

In the multifunctional ophthalmic test device as described above, as a schematic view is shown in FIG. 7(A), the camera device 30 treats, as an imaging range, a region including the eyes of the test subject who puts the chin on the viewpoint fixing mechanism 5. A compact CCD camera can be used as this camera device 30.

This camera device 30 is mounted on the vicinity of the outside of the hemispherical screen 1 in a fixed state thereto. As shown in FIG. 7(B), an eye portion image 200 acquired by this camera device 30 includes eye images 201 of the test subject. This eye portion image of the test subject is extracted by image processing such as edge extraction processing and pattern recognition. The multifunctional ophthalmic test device obtains an in-image position (eye position) of the eye images 201 acquired by the camera device 30. Then, the multifunctional ophthalmic test device calculates the viewpoint position with respect to the hemispherical screen 1 from the in-image position of the eye images 201, which is detected from the eye images 201, an attached position of the camera device 30, an attached orientation of the camera device 30, and a placed position of the chin on the viewpoint fixing mechanism 5. That is to say, the actual imaging range imaged by the camera device 30 is set by the attached position of the camera device 30 and the attached orientation of the camera device 30, and the actual position of the viewpoint can be calculated from the position of the eye images 201 in the eye portion image 200. Moreover, the placed position of the chin on the viewpoint fixing mechanism 5 is varied, whereby the position of the eye images 201 is varied. Hence, the viewpoint position is determined in consideration of also the placed position of the chin on the viewpoint fixing mechanism 5. Then, on the assumption that a line of sight is located on a horizontal line from the eye position to the hemispherical screen 1 concerned, an attention position on the hemispherical screen 1 when seen from the viewpoint position of the test subject can be calculated.

Moreover, desirably, as shown in FIG. 7(C), the multifunctional ophthalmic test device performs infrared radiation for the test subject, and detects the viewpoint position of the test subject from an image detected by a corneal reflex of the eyes. Here, in the case where an infrared ray is radiated toward the eyes of the test subject, the infrared ray concerned is reflected by the corneas of the eyes, and as shown in FIG. 7(D), the camera device 30 can image an eye portion image 200 including the eye images 201 and corneal reflex images 202. Then, the multifunctional ophthalmic test device can detect positions of the corneal reflex images 202 as the viewpoint position. In such a way, the multifunctional ophthalmic test device can obtain a more accurate viewpoint position than in the case of detecting the viewpoint position by only the eye images 201.

Note that an imaging direction of the camera device 30 may be made manually variable while the test subject is seeing a display that displays a video of the camera device 30, or from a relationship between the position of the chin support of the viewpoint fixing mechanism 5 and a usual size of a face, and the like, the eye position of the subject may be estimated and fixed. Moreover, for the image processing for obtaining the viewpoint position by using the eye images 201 and the cornea reflection images 202, which are contained in the eye portion image 200, there can be adopted a method of determining the presence of the eye images 201 in the case where circular images are present in the eye portion image 200.

In accordance with the multifunctional ophthalmic test device as described above, the camera device 30 is installed on the hemispherical screen 1. In such a way, in the multifunctional ophthalmic test device, even if the optometrist does not confirm the viewpoint position during the ophthalmic test by confronting the test subject or looking at the viewpoint position from aside, the optometrist can grasp the viewpoint position of the test subject, and can concentrate on the ophthalmic test. Moreover, restrictions on a positional relationship between the optometrist and the test subject are eliminated.

Moreover, detection accuracy of the viewpoint position may only indicate, for example, whether or not the viewpoint position is present at a place prescribed in advance with respect to the hemispherical screen 1. In order to represent this accuracy of the viewpoint position, the viewpoint position at present may be represented by using a numeric value and the like. Detection processing of the viewpoint position may be performed in real time or for each fixed time, and may be updated at the time when there occurs some trigger such as a button at the starting time of the ophthalmic test, and so on.

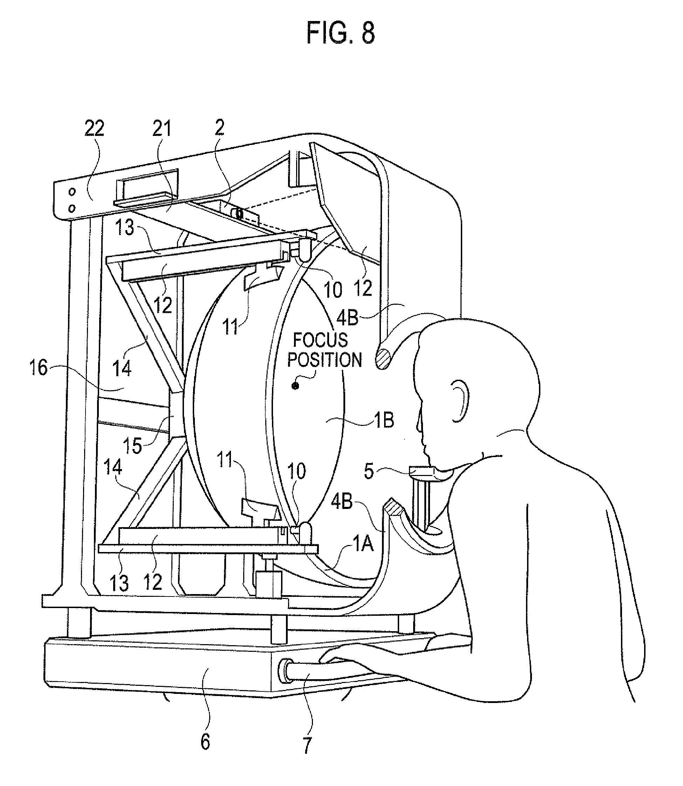

The viewpoint position is obtained as described above, whereby, for example as shown in FIG. 8, the multifunctional ophthalmic test device can display (present) the mark or the pointer, which indicates the viewpoint position. In order to display this mark, for example, image data indicating the arithmetically operated viewpoint position is supplied to the projector 2, and is projected onto the hemispherical screen 1. In such a way, the viewpoint position at present is presented to the test subject, and the test subject can be allowed to confirm whether or not the attention position seen from the viewpoint position concerned is suitable for the ophthalmic test, and then to move the viewpoint position, whereby an appropriate ophthalmic test can be performed.

Note that, as a display method of the mark, not only the mark corresponding to the viewpoint position at present is displayed, but also a distance to a target fixation point (viewpoint position), a method (up, down, left and right) of moving the chin, and the like can be displayed. Moreover, a desirable viewpoint position may be outputted by voice.

Moreover, the multifunctional ophthalmic test device can also include, as imaging means, a plurality of the camera devices 30, each of which treats the eye portion of the test subject as an imaging range, and can also detect the viewpoint position of the test subject by the CPU as the viewpoint position detecting means by using a plurality of the eye portion images 200 imaged by the plurality of camera devices 30. The multifunctional ophthalmic test device as described above can obtain the accurate viewpoint position by the CPU by using the plurality of eye portion images 200 imaged by the plurality of camera devices 30. For example, each viewpoint position can be arithmetically operated based on the eye images 201 contained in each of the eye portion images 200, and an average value of the plurality of viewpoint positions can be arithmetically operated.

Hence, in accordance with this multifunctional ophthalmic test device, even if the line of sight of the test subject treats, as the attention position, an arbitrary position on the wide hemispherical screen 1, the viewpoint position for seeing the attention position concerned can be arithmetically operated accurately. Moreover, even if the viewpoint position shifts from the target for performing the ophthalmic test, this multifunctional ophthalmic test device can accurately grasp the viewpoint position concerned that moves over a wide range, and can also arithmetically operate the attention position on the hemispherical screen 1, which corresponds to the viewpoint position concerned.

Note that, by using the plurality of camera devices 30, it becomes unnecessary to move orientations of the camera devices 30. Even if camera devices 30, each of which has a narrow imaging range, are used, the multifunctional ophthalmic test device can arithmetically operate the accurate viewpoint position by allowing the camera devices 30 to share a wide imaging range.

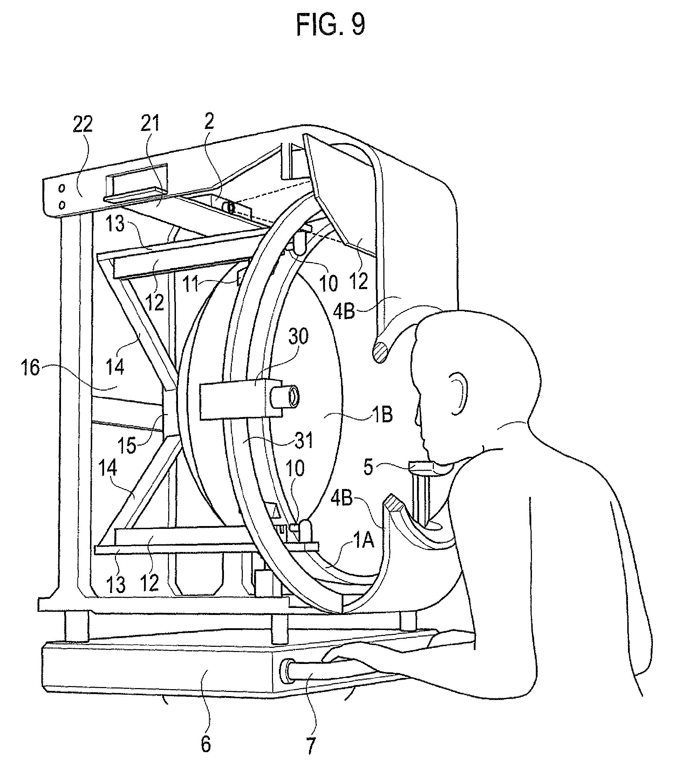

In place of including the plurality of camera devices 30 as described above, as shown in FIG. 9, the multifunctional ophthalmic test device may include, as the imaging means, a slide guide portion 31 provided along an outer circumference of the deep-bottom hemispherical screen 1A, and a camera device 30 that moves along the slide guide portion 31. In order to move the camera device 30 along the slide guide portion 31, the multifunctional ophthalmic test device includes a drive motor (not shown).

While being moved along the slide guide portion 31, the camera device 30 images a plurality of the eye portion images 200. The plurality of eye portion images 200 include the one in which the eye portions of the test subject are imaged from above, the one in which the eye portions of the test subject are imaged from aside, and the one in which the eye portions of the test subject are imaged from below. Then, the multifunctional ophthalmic test device can detect the viewpoint position of the test subject by using the plurality of eye portion images 200 in a similar way to the above-mentioned multifunctional ophthalmic test device, and from the viewpoint position concerned, can arithmetically operate the attention position on the hemispherical screen 1.

In accordance with the multifunctional ophthalmic test device as described above, the viewpoint position can be accurately detected even if a large number of the camera devices 30 are not used, and an amount of information on the eye portion images 200, cost power consumption of the device, and the like can be suppressed. Note that the camera device 30 may be driven in a panning direction or a tilting direction on the slide guide portion 31 in addition to the operation of being slid on the slide guide portion 31.

Moreover, another multifunctional ophthalmic test device may move the viewpoint position of the test subject with reference to the viewpoint position detected by the CPU as the viewpoint position detecting means. The multifunctional ophthalmic test device presets a desired viewpoint position. Then, in the case where the detected viewpoint position shifts from the desired viewpoint position concerned, the multifunctional ophthalmic test device drives the chin support in the viewpoint fixing mechanism 5 and a surface of a seat seated by the test subject so as to move the viewpoint position up and down or right and left. As described above, the multifunctional ophthalmic test device moves the viewpoint position to the viewpoint position desired for the ophthalmic test, whereby the ophthalmic test can be given to the test subject in an appropriate state.

Note that movement timing of the viewpoint position may be updated in real time or for each fixed time, and may be updated at the time when there occurs some trigger such as the button at the starting time of the ophthalmic test, and so on. Moreover, the multifunctional ophthalmic test device can preset moving speed and adjustment accuracy for adjusting the chin support and a chair in the viewpoint fixing mechanism 5, and can perform the movement of the viewpoint position. Furthermore, in the case of largely moving the viewpoint position, or in the case where there is a possibility that the test subject may contact the multifunctional ophthalmic test device, the multifunctional ophthalmic test device can discontinue the movement of the viewpoint position. Moreover, the multifunctional ophthalmic test device can control the orientation of the camera device 30 concerned in conjunction with the movement of the viewpoint position, which corresponds to the motion of the chin support, and can also thereby detect the corneal reflex images 202 by the corneal reflex more efficiently.

Moreover, in the multifunctional ophthalmic test device, the CPU as the viewpoint position detecting means detects a right eye viewpoint position and left eye viewpoint position of the test subject by using the eye portion image 200 imaged by the camera device 30. Then, desirably, the multifunctional ophthalmic test device detects the right eye viewpoint position and the left eye viewpoint position one more time in the case where a distance between the viewpoint positions is abnormal.

The multifunctional ophthalmic test device presets a usual inter-eye distance as a threshold value, and compares this threshold value with the detected distance between the right eye viewpoint position and the left eye viewpoint position. This threshold value is preset, for example, at approximately 10 cm. As a result, in the case where the distance between the right eye viewpoint position and the left eye viewpoint position exceeds the threshold value, the eye portion image 200 is imaged one more time. At this time, the multifunctional ophthalmic test device performs an operation of changing the position of the camera device 30, an operation of increasing the number of eye portion images 200, an operation of radiating the infrared ray, and the like, and performs a re-measurement that is capable of detecting the viewpoint position with high reliability. Then, in the case where the distance between the right eye viewpoint position and the left eye viewpoint position is smaller than the threshold value, the detection of the viewpoint position is performed by using the eye portion image 200 concerned. In such a way, the multifunctional ophthalmic test device can detect the viewpoint position of the test subject by using the eye portion image 200 imaged in a normal state, and can thereby perform the ophthalmic test.

Still further, in the multifunctional ophthalmic test device, the CPU as the viewpoint position detecting means detects the binocular viewpoint position, right eye viewpoint position and left eye viewpoint position of the test subject by using the eye portion image 200 imaged by the camera device 30. Then, the CPU may correct the binocular viewpoint position based on the right eye viewpoint position concerned and the left eye viewpoint position concerned.

For example, the multifunctional ophthalmic test device receives a degree of a squint of a patient in advance, and corrects the binocular viewpoint position, which is obtained from the right eye viewpoint position and the left eye viewpoint position, in response to the degree of the squint. In such a way, while the attention position obtained from the right eye viewpoint position and the left eye viewpoint position shifts, the multifunctional ophthalmic test device can correct the binocular viewpoint position, and can also thereby correct the attention position when the hemispherical screen 1 is seen from the binocular viewpoint position thus corrected. As described above, in accordance with the multifunctional ophthalmic test device, even if the test subject is the patient of the squint, the binocular viewpoint position of the patient concerned can be obtained accurately, and the attention position corresponding to the binocular viewpoint position concerned can be thereby obtained accurately.

As described above, in accordance with the multifunctional ophthalmic test device, the viewpoint positions of the test subject are detected, whereby the ophthalmic test can be performed while measuring the attention position on the hemispherical screen 1, which corresponds to the viewpoint positions concerned. Hence, in accordance with this multifunctional ophthalmic test device, even if the hemispherical screen 1 with a wide visual field is used, it can be recorded how the result of the ophthalmic test is obtained, that is, from which viewpoint the test subject pays attention on which position on the hemispherical screen 1. This can contribute to enhancement of the reliability of ophthalmic test result, and the like.

Moreover, heretofore, it has been necessary to manually adjust the viewpoint position of the test subject, and moreover, in the case where the viewpoint position has shifted after being manually adjusted, the viewpoint position has not been able to be automatically adjusted to a designated viewpoint position. As opposed to this, in accordance with the multifunctional ophthalmic test device, the viewpoint position and attention position of the test subject are arithmetically operated automatically, whereby the viewpoint fixing mechanism 5 can be driven so as to obtain the desired viewpoint position, and a mark that prompts the test subject to see the desired attention position can be displayed on the hemispherical screen 1.

Furthermore, in accordance with this multifunctional ophthalmic test device, the ophthalmic test can also be performed while allowing the stereopsis in such a manner that the video is projected with a wide visual field onto the hemispherical screen 1, and accordingly, the ophthalmic test in the daily vision can be performed. That is to say, at the time of performing the ophthalmic test in the daily vision, which is performed without fixing the viewpoint position, the ophthalmic test result can be acquired while recording in real time the unfixed viewpoint position and the attention position at the time of the viewpoint position concerned, and reliability of the ophthalmic test result in the daily vision concerned can be enhanced.

### INDUSTRIAL APPLICABILITY

The present invention can be applied to the ophthalmic tests such as the visual field test, the binocular vision test, the binocular visual function test (simultaneous perception/flat fusion/stereopsis), the position of eyes/eye movement test, and the aniseikonia test.

## Claims

1. A multifunctional ophthalmic test device comprising:
a deep-bottom hemispherical screen and a shallow-bottom hemispherical screen, which are different in depth from each other;
projecting means for projecting an ophthalmic test image on either of the deep-bottom hemispherical screen and the shallow-bottom hemispherical screen; and
driving means for driving, in accordance with a type of an implemented ophthalmic test, either of the deep-bottom hemispherical screen and the shallow-bottom hemispherical screen so that the ophthalmic test image can be projected by the projecting means onto either of the deep-bottom hemispherical screen and the shallow-bottom hemispherical screen.

2. The multifunctional ophthalmic test device according to claim 1, further comprising:
ophthalmic test type inputting means for inputting the type of the implemented ophthalmic test,
wherein the driving means drives the deep-bottom hemispherical screen to a position, onto which the ophthalmic test image is projected by the projecting means, in a case where the type of the ophthalmic test, the type being inputted by the ophthalmic test type inputting means, is a visual field test, and drives either of the shallow-bottom hemispherical screen and the deep-bottom hemispherical screen to the position, onto which the ophthalmic test image is projected by the projecting means, in a case where the type of the ophthalmic test, the type being inputted by the ophthalmic test type inputting means, is other than the visual field test.

3. The multifunctional ophthalmic test device according to claim 2, wherein the projecting means includes a left eye-ready projection unit and a right eye-ready projection unit, and in a case where the type of the ophthalmic test, the type being inputted by the ophthalmic test type inputting means, is one to present separate videos for a left eye and a right eye, the projecting means performs either of an operation of projecting a stereoscopic video by the left eye-ready projection unit and the right eye-ready projection unit, and an operation of projecting different videos for the left eye and the right eye by the left eye-ready projection unit and the right eye-ready projection unit.

4. The multifunctional ophthalmic test device according to claim 2, further comprising:
index mark displaying means for displaying an index mark for the visual field test on the deep-bottom hemispherical screen by the projecting means in a case of performing the visual field test.

5. The multifunctional ophthalmic test device according to any one of claims 1 to 4,
wherein an entirety of a hemispherical screen with an arbitrary depth is the deep-bottom hemispherical screen, a bottom portion of the hemispherical screen is the shallow-bottom hemispherical screen, and a portion of the shallow-bottom hemispherical screen in the hemispherical screen and a portion other than the shallow-bottom hemispherical screen in the hemispherical screen are composed separately from each other, and
the driving means drives the portion other than the shallow-bottom hemispherical screen in the hemispherical screen, and locates either of the deep-bottom hemispherical screen and the shallow-bottom hemispherical screen at a position onto which the ophthalmic test image is projected by the projecting means.

6. The multifunctional ophthalmic test device according to any one of claims 1 to 5, further comprising:
a planer screen as any of a screen for a vision test, a screen for a binocular vision test, and a screen for an aniseikonia test.

7. The multifunctional ophthalmic test device according to any one of claims 1 to 6, further comprising:
peripheral brightness adjusting means for adjusting brightness of a periphery of either of the deep-bottom hemispherical screen and the shallow-bottom hemispherical screen.

8. The multifunctional ophthalmic test device according to any one of claims 1 to 7, further comprising:
imaging means for imaging an eye portion of a test subject subjected to the ophthalmic test by the ophthalmic test image projected onto either of the deep-bottom hemispherical screen and the shallow-bottom hemispherical screen; and
viewpoint position detecting means for detecting a viewpoint position of the test subject by using an eye portion image imaged by the imaging means.

9. The multifunctional ophthalmic test device according to claim 8,
wherein the imaging means is composed of a plurality of camera devices, each treating the eye portion of the test subject as an imaging range, and
the viewpoint position detecting means detects the viewpoint position of the test subject by using a plurality of the eye portion images imaged by the plurality of cameras.

10. The multifunctional ophthalmic test device according to claim 8,
wherein the imaging means includes: a slide guide portion provided along an outer circumference of the deep-bottom hemispherical screen; and a camera device that moves along the slide guide portion,
a plurality of the eye portion images are imaged while moving the camera device along the slide guide portion, and
the viewpoint position detecting means detects the viewpoint position of the test subject by using the plurality of eye portion images.

11. The multifunctional ophthalmic test device according to any one of claims 8 to 10, further comprising:
presenting means for presenting, on either of the deep-bottom hemispherical screen and the shallow-bottom hemispherical screen, a mark representing the viewpoint position of the test subject, the viewpoint position being detected by using the eye portion image imaged by the imaging means.

12. The multifunctional ophthalmic test device according to any one of claims 8 to 10, further comprising:
moving means for moving the viewpoint position of the test subject with reference to the viewpoint position detected by the viewpoint position detecting means.

13. The multifunctional ophthalmic test device according to any one of claims 8 to 10, wherein the viewpoint position detecting means detects a right eye viewpoint position and left eye viewpoint position of the test subject by using the eye portion image imaged by the imaging means, and detects the viewpoint position one more time in a case where a distance between the right eye viewpoint position and the left eye viewpoint position is abnormal.

14. The multifunctional ophthalmic test device according to any one of claims 8 to 10, wherein the viewpoint position detecting means detects a binocular viewpoint position, right eye viewpoint position and left eye viewpoint position of the test subject by using the eye portion image imaged by the imaging means, and corrects the binocular viewpoint position based on the right eye viewpoint position and the left eye viewpoint position.
